# EUROPÄISCHE PATENTSCHRIFT

(11) **EP 0 570 355 B1**
(45) Veröffentlichungstag und Bekanntmachung des Hinweises auf die Patenterteilung: **30.12.1998**
(21) Anmeldenummer: 93890098.2
(22) Anmeldetag: 12.05.1993
(51) Int. Cl.: C12Q 1/56, G01N 33/86

(54) **Verwendung von Prothrombinfragmenten**
Use of prothrombin fragments
Utilisation des fragments prothrombines

(30) Priorität: 15.05.1992 AT 1000/92
(43) Veröffentlichungstag der Anmeldung: 18.11.1993
(73) Patentinhaber: IMMUNO Aktiengesellschaft, A-1221 Wien (AT)
(72) Erfinder: Lang, Hartmut, Dr., A-1230 Wien (AT); Moritz, Berta, Dr., A-1030 Wien (AT)
(74) Vertreter: Weinzinger, Arnulf, Dipl.-Ing.

(56) Entgegenhaltungen:
- EP-A- 49 877
- EP-A- 420 332
- EP-A- 442 843
- WO-A-92/07954
- JOURNAL OF BIOLOGICAL CHEMISTRY Bd. 266, Nr. 6, 25. Februar 1991, BALTIMORE, MD US Seiten 4017 - 4022 P. N. M. TIJBURG ET AL. 'Formation of meizothrombin as intermediate in factor Xa-catalysed prothrombin activation on endothelial cells.'
- JOURNAL OF BIOLOGICAL CHEMISTRY Bd. 265, Nr. 18, 25. Juni 1990, BALTIMORE, MD US Seiten 10693 - 10701 M. F. DOYLE ET AL. 'Multiple active forms of thrombin.'
- CHEMICAL ABSTRACTS, vol. 98, no. 3, 17. Januar 1983, Columbus, Ohio, US; abstract no. 13567, E. BRIET ET AL. 'Cleavage and activation of human prothrombin by Echis carinatus venom.' Seite 250 ;Spalte 1 ;

## Beschreibung

Die Erfindung betrifft eine neue Verwendung von Prothrombinfragmenten, insbesondere von Meizothrombin, Meizothrombin (desF1) oder Mischungen daraus.

Die Bestimmung von Thrombinsubstraten durch Aktivierung mittels Thrombin stellt eine wichtige Methodik zur Charakterisierung von Blut- und Plasmaprodukten dar. Beispielsweise werden Konzentrationen bzw. Aktivitäten von Fibrinogen, Faktor V, Faktor VIII oder Protein C mittels Aktivierung durch Thrombin standardmäßig in Plasma-hältigen Proben bestimmt.

Thrombin ist ein 36 kD-Protein, das eine zentrale Rolle in der Blutgerinnungskaskade spielt, denn seine Hauptfunktion ist die Umwandlung von Fibrinogen in Fibrin, das unter Mithilfe des ebenfalls von Thrombin aktivierten Faktors XIII ein Fibrinnetz ausbildet.

Darüberhinaus aktiviert Thrombin auch Blutgerinnungsfaktoren, wie Faktor V, Faktor VIII und Protein C, und bewirkt die Thrombozytenaggregation. Eine Reihe anderer Proteine wie Fibronectin, Thrombospondin und Apolipoprotein, verschiedene Kollagene, Laminin, etc. (siehe Tabelle 1 in Stocker, Sem.Thr.Hem. 17 (2) S. 114 (1991)) können ebenfalls von Thrombin gespalten werden. Derartige Proteine werden in der folgenden Beschreibung als "Thrombinsubstrate" bezeichnet.

Aufgrund seiner wichtigen Stellung in der Blutgerinnungskaskade wird die Wirkung des Thrombins durch Aktivitätseffektoren bzw. Inhibitoren in vivo sehr genau reguliert.

Insbesondere die Inhibitoren sind aber bei der Bestimmung von Thrombinsubstraten störend, da die Inhibitoren in einer Probe die Ergebnisse verfälschen bzw. vor der Bestimmung eine viel größere, überschüssige Thrombinmenge zugesetzt werden muß, um die vorhandenen Inhibitoren zu überspielen.

Ein wichtiger Thrombin-Inhibitor ist beispielsweise Heparin zusammen mit Antithrombin III. Heparin ist praktisch in allen Blutproben enthalten, wenn Blutspenden unmittelbar nach der Entnahme zur Verhinderung einer vorzeitigen Gerinnung heparinisiert, d.h. mit Heparin versetzt werden oder wenn der Patient Heparin z.B. zur Antikoagulationstherapie erhalten hat.

Das eigentliche Thrombin-inhibierende Agens ist Antithrombin III. Heparin potenziert den Effekt von Antithrombin III.

Bei einer aus einer Heparin-hältigen Blut- oder Plasmaprobe besteht also immer das Risiko, verfälschte Ergebnisse bei Thrombinkonzentrations-, Thrombinsubstrataktivierungs- oder Kinetikbestimmungen zu erhalten, sofern sie nicht auf aufwendige Weise vorher vom Heparin befreit oder das Heparin neutralisiert worden ist.

Dadurch wird ein zusätzlicher Verfahrensschritt notwendig, was für diagnostische Verfahren, die für Routinezwecke möglichst einfach durchzuführen sein sollten, wenig vorteilhaft ist. Jeder zusätzliche Verfahrensschritt bedeutet nicht nur einen höheren Arbeitsaufwand, sondern ist auch eine weitere potentielle Fehlerquelle des Bestimmungsverfahrens.

Thrombin wird im Blut aus einem inaktiven Vorläuferprotein, dem Prothrombin, über mehrere Zwischenstufen zum reifen Enzym gebildet. Das reife Enzym besteht aus zwei Ketten (A- und B-Kette), die durch eine Disulfid-Brücke miteinander verbunden sind.

Meizothrombin oder Meizothrombin (desF1) sind solche Prothrombinfragmente, die durch nicht vollständige Aktivierung von Prothrombin zu Thrombin entstanden sind (siehe Abbildung 1 in Doyle et al., J.Biol.Chem. 265 (18), 10693-10701 (1990)). Meizothrombin (desF1) entsteht durch Aktivierung von Prothrombin in Abwesenheit eines Thrombininhibitors. Dabei wird zuerst Meizothrombin gebildet, welches nach Abspaltung des Fragmentes F1 zu Meizothrombin (desF1) proteolytisch abgebaut wird. Das Molekulargewicht reduziert sich damit von ungefähr 72 kD auf etwa 48 kD. Diese Fragmente gelten als instabil, weil sie autokatalytisch degradieren, beispielsweise wird Meizothrombin rasch durch Autokatalyse in α-Thrombin umgewandelt. Meizothrombin und Meizothrombin (desF1) können auch durch Einwirkung des Schlangengifts Ecarin auf Prothrombin hergestellt werden.

Versuche von Doyle et al. haben gezeigt, daß bovines Meizothrombin im Gegensatz zu Prothrombin eine Proteaseaktivität aufweist, die im Vergleich zu Thrombin sehr gering ist.

Meizothrombin ist zwar in der Lage, mit einer geringen Rate z.B. Fibrinogen in Fibrin zu spalten oder Protein C zu aktivieren, jedoch wird es von Heparin nicht inhibiert bzw. ist der Inhibitonseffekt um Größenordnungen kleiner (Stocker & Müller, Thrombosis and Haemostasis 65 (6), Abstract 855 (1991)). Es hat sich aber auch gezeigt, daß humane Prothrombin-Fragmente wesentlich instabiler sind als beispielsweise bovine.

Es ist aber auch bekannt, daß einige Schlangengifte Thrombinsubstrate spalten. Andersson (Haemostasis 1, 31-43 (1972)) beschreibt eine "thrombin like activity" mehrerer Schlangengifte und drückt diese analog zu Thrombin in NIH-Einheiten/ml aus. Die Verwendung von Schlangengiften im Rahmen der Diagnostik mit humanen Proteinen hat jedoch den Nachteil der unspezifischen Wechselwirkungen (Intraspezies-Interaktionen). Man ist daher vor allem bei der Entwicklung von diagnostischen Verfahren zur Bestimmung von humanen Faktoren bemüht, nur Säugetierproteine, vorzugsweise humane Proteine, einzusetzen. Eine wesentliche Voraussetzung für ein Bestimmungsverfahren ist die eindeutige Reaktion der eingesetzten Proteine.

Der Erfindung liegt die Aufgabe zugrunde, unter Vermeidung von durch Inhibitoren verursachten Fehlerquellen eine neue Verwendung von Prothrombin-Fragmenten zur Verfügung zu stellen. Damit soll die Erfindung eine einfache und genaue Methode zur Bestimmung von Thrombinsubstraten ermöglichen.

Gegenstand der Erfindung ist somit die Verwendung von Prothrombinfragmenten, vorzugsweise humanen Prothrombinfragmenten, mit einer "thrombin like activity", nämlich von Meizothrombin, Meizothrombin (desFl) oder Mischungen daraus, für diagnostische Zwecke zur Bestimmung von Thrombinsubstraten.

Es hat sich herausgestellt, daß diese Fragmente in der Lage sind, Substrate, die von Thrombin umgesetzt werden können, ebenfalls zu spalten. Eine "thrombin-like activity" bedeutet hier eine Proteaseaktivität, die Thrombinsubstrate zu spalten vermag, deren Reaktionskinetik und Reaktionsparameter, wie pH- oder Ionenstärkeoptima, Inhibitoren, Effektoren, allosterische Wechselwirkungen, usw. aber nicht unbedingt denen von Thrombin gleichen müssen.

Die erfindungsgemäße Verwendung hat den Vorteil, daß die "thrombin like activity" der genannten Substanzen aufgrund ihrer geringen Affinität zu Antithrombin III zum Unterschied von der Thrombin-Aktivität Heparin-unempfindlich ist. Eine diagnostische Bestimmung unter Verwendung der genannten Plasmaproteine ist also unabhängig vom Heparingehalt einer Probe und somit in einfacher Weise durchzuführen.

Demnach können Thrombin-Substrate, wie Fibrinogen, Faktor V, Faktor VIII, Faktor XIII und Protein C, durch Aktivierung mit einem Reagens, enthaltend die genannten Prothrombinfragmente, in direkter Weise bestimmt werden.

Erfindungsgemäß können nicht nur Thrombinsubstrate bestimmt werden, sondern auch Substanzen, die mit erfindungsgemäß aktivierten Thrombinsubstraten in irgendeiner Weise wechselwirken.

Das Reagens kann also zur indirekten Bestimmung von Faktoren der Gerinnungskaskade über die aktivierten Thrombinsubstrate verwendet werden. Faktor VIII kann z.B. einem Reagens zugesetzt und in situ aktiviert werden. Damit wird die Bestimmung von Faktor IX und darüber hinaus von Faktor X möglich.

Die Erfindung betrifft daher auch die Verwendung von Prothrombinfragmenten, vorzugsweise humanen Prothrombin-fragmenten, mit einer "thrombin like activity", nämlich Meizothrombin, Meizothrombin (desFl) oder Mischungen davon, zur Aktivierung von Thrombinsubstraten mit anschließender Bestimmung eines Plasmaproteins, welches mit dem aktivierten Thrombinsubstrat in direkter oder indirekter Weise wechselwirkt.

Erfindungsgemäß werden Meizothrombin oder Meizothrombin (desFl), vorzugsweise humanes Meizothrombin oder Meizothrombin (desFl), einzeln oder als Gemisch verwendet. Diese Substanzen können in standardisierter Weise zur Verfügung gestellt werden, beispielsweise durch kontrollierte Spaltung, Verwendung von Schlangengiften, usw., wobei sich überraschenderweise zeigte, daß sie eine für Diagnostika notwendige ausreichende Stabilität aufweisen.

Auch gentechnisch hergestellte Proteine, welche aufgrund ihrer Struktur eine Aktivität ähnlich der von Thrombin zeigen und im Vergleich zu Thrombin eine geringere Affinität zu Antithrombin III aufweisen, sind für die erfindungsgemäße Verwendung geeignet.

Die Erfindung umfaßt weiters ein Reagens enthaltend Meizothrombin, Meizothrombin (desFl) oder Mischungen daraus, zur Verwendung für diagnostische Zwecke zur Bestimmung von Thrombinsubstraten und zur Aktivierung von Thrombinsubstraten mit anschließender Bestimmung eines Plasmaproteins, welches mit dem aktivierten Thrombinsubstrat in direkter oder indirekter Weise wechselwirkt, beispielsweise Proteine der Gerinnungskaskade wie Faktor Xa und Faktor Va. Reagenzien, welche die genannten Prothrombin-Fragmente enthalten und erfindungsgemäß verwendet werden, sind gegebenenfalls lyophilisiert und enthalten gegebenenfalls weiters ein aktivierbares Thrombinsubstrat zur Verwendung für diagnostische Zwecke.

Die Erfindung kann auch ein Reagens, welches weiters ein oder mehrere Plasmaproteine, die aktiviert sein können, Ca²+-Ionen und gegebenenfalls Phospholipide enthält, oder ein Reagens, welches weiters ein Detektionsreagens enthält, umfassen.

Weiters läßt sich ein einfacher Testkit herstellen, der neben den beanspruchten Reagentien noch ein Detektionsreagens beinhaltet.

Der Testkit enthält mindestens
- ein Reagens enthaltend Meizothrombin, Meizothrombin (desFl) oder Mischungen daraus, oder
- ein Reagens enthaltend Meizothrombin, Meizothrombin (desF1) oder Mischungen daraus, welches weiters ein oder mehrere Plasmaproteine, die aktiviert sein können, enthält, und
- ein Detektionsreagens,
und kann vorzugsweise zusätzlich
- ein Thrombinsubstrat
enthalten.

Die Wahl der Detektionsmethode und damit des Detektionsreagens richtet sich natürlich nach der Natur der zu bestimmenden Substanz in der Probe. Beispielsweise wird die Aktivität von aktiviertem Protein C mit dem chromogenen Substrat S 2238 (Fa. Kabi) bestimmt; es kann die Gerinnungszeit bestimmt, oder es können markierte Substanzen eingesetzt werden.

Durch die folgenden Beispiele soll die Erfindung noch näher erläutert werden.

### Beispiele:

### 1. Herstellung von Meizothrombin und Molekulargewichtsverteilung

### 1.1 Herstellung von immobilisiertem Ecarin

Ecarin (Fa. Pentapharm) wurde in einer Konzentration von 250 E/ml Gel an BrCNaktivierte Sepharose (Fa. Pharmacia) nach der Methode von Axen et al. (1967) gebunden.

### 1.2 Herstellung von Meizothrombin

1 ml Gel wurde mit 4 E Prothrombin (aus Plasma gereinigt durch Adsorption an DEAE-Sephadex und anschließende weitere chromatographische Reinigung) in einem 0,02 M Na-Acetatpuffer, 0,15 M NaCl, pH 5,2, 1 Stunde bei Raumtemperatur inkubiert. Dann wurde das Produkt durch Filtration vom Gel abgetrennt.

### 1.3 Molekulargewichtsverteilung

Nach nativer SDS-PAGE und Immunoblotting (1. Antikörper: Anti-F.II, polyklonal vom Kaninchen; 2. Antikörper: Peroxidasekonjugierter Anti-Rabbit Antikörper von der Ziege) wurde die Molekulargewichtsverteilung bestimmt und eine Quantifizierung durchgeführt. Das Produkt weist folgende Molekulargewichtsverteilungen auf; "thrombin like activity": 67 bis 75 kD (31 %), 45 bis 50 kD (40 %). Weiters wurde gefunden: > 100 kD (7 %), 32 bis 38 kD (13 %), < 20 kD (9 %).

Reference: Axen R, Porath A.R., Ernback J.S., Nature 214, 1392-1404, 1967.

### 2. Fibrinogenbestimmung

### 2.1 Gerinnungszeit von Normalplasma

1 ml lyophilisiertes Normalplasma (Reference Plasma 100 %, Fa. Immuno) wurde in 1 ml Wasser mit oder ohne Heparinzusatz (0; 0,3; 0,6; 1,0 und 2,0 E/ml) gelöst. 200 µl des Plasmas wurden 2 min bei 37°C inkubiert. Danach wurde mit 200 µl einer Lösung von 5,8 NIH-analog E/ml Meizothrombin, hergestellt nach 1., oder mit 3,3 E/ml bzw. 10,0 NIH-E/ml Thrombin (Thrombin Reagent, Fa. Immuno) die Gerinnung ausgelöst. Die Zeit bis zur Clot-Bildung wurde mit einem Schnitger-Gross-Koagulometer (Fa. Amelung), gemessen. Aus der Tabelle 1 ist ersichtlich, daß die Gerinnungszeit der Thrombin-Ansätze durch den Heparingehalt der Probe verlängert wird, während die Wirkung von Meizothrombin von Heparin kaum beeinflußt wird.

| Gerinnungszeit (s) | | | | | |
|---|---|---|---|---|---|
| | Heparingehalt der Probe (E/ml) | | | | |
| | 0 | 0,3 | 0,6 | 1,0 | 2,0 |
| Thrombin (NIH-E/ml) | | | | | |
| 3,3 | 16,1 | 96,1 | >400 | >400 | >400 |
| 10 | 7,0 | 15,6 | 67,4 | >400 | >400 |

| Meizothrombin (NIH-analog E/ml) | | | | | |
|---|---|---|---|---|---|
| 5,8 | 5,5 | 6,1 | 7,6 | 8,0 | 8,6 |

### 2.2 Bezugskurve für Fibrinogen

Lyophilisiertes Normalplasma (siehe 2.1) wurde mit 1 ml destilliertem Wasser mit und ohne Zusatz von diversen Thrombin-Inhibitoren (je 1 E/ml von Antithrombin III, Heparin oder Antithrombin III/Heparin-Komplex (Atheplex®, Fa. Immuno)) gelöst und mit 0,1 M Phosphatpuffer (pH 7,5) unterschiedlich verdünnt. Davon wurden 100 µl Probe mit 50 µl desselben Puffers vermischt und 2 min bei 37°C inkubiert. Nach Zusatz von 150 µl einer Lösung von 10,5 NIH-analog E/ml Meizothrombin (Fa. Pentapharm) oder 5 NIH-E/ml Thrombin (Thrombin Reagent Fa. Immuno) wurde die Gerinnungszeit analog zu 2.1 bestimmt.

| Gerinnungszeit mit Thrombin (s) | | | | |
|---|---|---|---|---|
| | Inhibitorzusatz | | | |
| | kein Inhib. | Atheplex | ATIII | Heparin |
| Probe konz. | 17,1 | >300 | >350 | >300 |
| 1:2 | 21,6 | 61,6 | >350 | 50,4 |
| 1:4 | 27,6 | 37,1 | 101,6 | 36,1 |
| 1:8 | 41,6 | 38,6 | 54,6 | 48,9 |

| Gerinnungszeit mit Meizothrombin (s) | | | | |
|---|---|---|---|---|
| | Inhibitorzusatz | | | |
| | kein Inhib. | Atheplex | ATIII | Heparin |
| Probe konz. | 15,1 | 16,0 | 19,7 | 25,5 |
| 1:2 | 23,6 | 26,6 | 28,8 | 31,6 |
| 1:4 | 34,6 | 36,6 | 37,1 | 39,1 |
| 1:8 | 56,1 | 57,6 | 54,0 | 60,6 |

### 3. Meizothrombin in einem Diagnostikum als Aktivator der Gerinnungskaskade

Faktor VIII in einem Normalplasma (siehe 2.1) wurde nach Zusatz von 0; 1; 2; 5 oder 10 E Heparin/ml mit Meizothrombin, hergestellt nach 1., (0; 3 oder 5 E/ml) aktiviert und indirekt über Faktor Xa und einem chromogenen Substrat bestimmt. Dazu wurde der Test Immunochrom FVIII:C® (IMMUNO AG) laut Herstellerangaben verwendet, wobei dem Reagens A Meizothrombin zugesetzt wurde und Reagens B ohne Polybren verwendet wurde. Dieser Test enthält Thrombin, wodurch ein direkter Vergleich von Meizothrombin und Thrombin zur Bestimmung aktivierter Gerinnungsfaktoren im Testsystem möglich ist. Die folgende Tabelle zeigt, daß in Anwesenheit von Meizothrombin die Aktivierung und Bestimmung des Faktor VIII durch den Heparingehalt des Plasmas weniger beeinflußt wird.

| Heparin (E/ml) | 0 | 1 | 2 | 5 | 10 |
|---|---|---|---|---|---|
| Meizothrombin (NIH-analog E/ml) | | | | | |
| | Farbentwicklung (%) | | | | |
| 0 | 100 | 99 | 95,6 | 80 | 52,1 |
| 3 | 100 | 99 | 97,4 | 87,8 | 73,3 |
| 5 | 100 | 100 | 98 | 92,2 | 80,1 |

## Patentansprüche

1. Verwendung von Meizothrombin, Meizothrombin (desF1) oder Mischungen daraus, für diagnostische Zwecke zur Bestimmung von Thrombinsubstraten.

2. Verwendung nach Anspruch 1, dadurch gekennzeichnet, daß humanes Meizothrombin, Meizotrhombin (desF1) oder Mischungen daraus eingesetzt werden.

3. Verwendung von Meizothrombin, Meizothrombin (desF1) oder Mischungen daraus, zur Aktivierung von Thrombinsubstraten mit anschließender Bestimmung eines Plasmaproteins, welches mit dem aktivierten Thrombinsubstrat in direkter oder indirekter Weise wechselwirkt.

4. Verwendung nach Anspruch 3, dadurch gekennzeichnet, daß humanes Meizothrombin, Meizotrhombin (desF1) oder Mischungen daraus eingesetzt werden.

5. Reagens, enthaltend Meizothrombin, Meizothrombin (desFl) oder Mischungen daraus, welches weiters ein oder mehrere Plasmaproteine, die aktiviert sein können, Ca²⁺-Ionen und gegebenenfalls Phospholipide enthält.

6. Reagens nach Anspruch 5, dadurch gekennzeichnet, daß es humanes Meizothrombin, Meizothrombin (desF1) oder Mischungen daraus enthält.

7. Reagens nach Anspruch 5 oder 6, welches weiters ein Detektionsreagens enthält.

8. Reagens, enthaltend Meizothrombin, Meizothrombin (desFl) oder Mischungen daraus, welches weiters ein Detektionsreagens enthält.

9. Reagens nach Anspruch 8, dadurch gekennzeichnet, daß es humanes Meizothrombin, Meizothrombin (desF1) oder Mischungen daraus enthält.

10. Testkit, mindestens enthaltend
- ein Reagens nach einem der Ansprüche 5 oder 6,
- ein Detektionsreagens.

11. Testkit nach Anspruch 10, weiters enthaltend
- ein Thrombinsubstrat.

12. Testkit, mindestens enthaltend
- ein Reagens nach einem der Ansprüche 7 bis 9,
- ein Thrombinsubstrat.

## Claims

1. The use of meizothrombin, meizothrombin (desF1) or mixtures thereof for diagnostic purposes for assaying thrombin substrates.

2. The use according to claim 1, characterised in that human meizothrombin, meizothrombin (desF1) or mixtures thereof are utilized.

3. The use of meizothrombin, meizothrombin (desF1) or mixtures thereof for activating thrombin substrates followed by a determination of a plasma protein that interacts directly or indirectly with the activated thrombin substrate.

4. The use according to claim 3, characterised in that human meizothrombin, meizothrombin (desF1) or mixtures thereof are utilized.

5. A reagent comprising meizothrombin, meizothrombin (desF1) or mixtures thereof, further comprising one or more plasma proteins which may be activated, Ca²⁺ ions and optionally phospholipids.

6. A reagent according to claim 5, characterised in that it comprises human meizothrombin, meizothrombin (desF1) or mixtures thereof.

7. A reagent according to claim 5 or 6, further comprising a detection reagent.

8. A reagent comprising meizothrombin, meizothrombin (desF1) or mixtures thereof, further comprising a detection reagent.

9. A reagent according to claim 8, characterised in that it comprises human meizothrombin, meizothrombin (desF1) or mixtures thereof.

10. A test kit, comprising at least
- a reagent according to any one of claims 5 or 6,
- a detection reagent.

11. A test kit according to claim 10, further comprising
- a thrombin substrate.

12. A test kit, comprising at least
- a reagent according to any one of claims 7 to 9,
- a thrombin substrate.

## Revendications

1. Utilisation de meizothrombine, de meizothrombine (desF1) ou de leurs mélanges à des fins diagnostiques pour la détermination de substrats de la thrombine.

2. Utilisation selon la revendication 1, caractérisée en ce qu'on utilise de la meizothrombine, de la meizothrombine (desF1) humaines ou leurs mélanges.

3. Utilisation de meizothrombine, de meizothrombine (desF1) ou de leurs mélanges pour l'activation de substrats de la thrombine avec détermination subséquente d'une protéine plasmatique qui interagit de façon directe ou indirecte avec le substrat de thrombine activé.

4. Utilisation selon la revendication 3 caractérisée en ce qu'on utilise de la meizothrombine, de la meizothrombine (desF1) humaines ou leurs mélanges.

5. Réactif contenant de la meizothrombine, de la meizothrombine (desF1) ou leurs mélanges, lequel contient en outre une ou plusieurs protéines plasmatiques susceptibles d'être activées, des ions Ca²⁺ et le cas échéant des phospholipides.

6. Réactif selon la revendication 5, caractérisé en ce qu'il contient de la meizothrombine, de la meizothrombine (desF1) humaines ou leurs mélanges.

7. Réactif selon la revendication 5 ou 6, lequel contient en outre un réactif de détection.

8. Réactif contenant de la meizothrombine, de la meizothrombine (desF1) ou leurs mélanges, lequel contient en outre un réactif de détection.

9. Réactif selon la revendication 8, caractérisé en ce qu'il contient de la meizothrombine, de la meizothrombine (desF1) humaines ou leurs mélanges.

10. Kit de dosage, contenant au moins
- un réactif selon l'une des revendications 5 ou 6,
- un réactif de détection.

11. Kit de dosage selon la revendication 10, contenant en outre :
- un substrat de la thrombine.

12. Kit de dosage, contenant au moins
- un réactif selon une des revendications 7 à 9,
- un substrat de la thrombine.
